# EUROPEAN PATENT SPECIFICATION

(11) **EP 0 946 967 B1**
(45) Date of publication and mention of the grant of the patent: **01.09.2004**
(21) Application number: 98942951.9
(22) Date of filing: 18.09.1998
(51) Int. Cl.: H01J 61/00

(54) **LIGHT-EMITTING APPARATUS**
LICHTEMITTIERENDES GERÄT
APPAREIL PHOTOEMETTEUR

(30) Priority: 19.09.1997 WO PCT/IB97/01123
(43) Date of publication of application: 06.10.1999
(73) Proprietor: International Business Machines Corporation, Armonk, N.Y. 10504 (US)
(72) Inventor: GIMZEWSKI, James, K., CH-8803 Rueschlikon (CH); GUERET, Pierre, L., CH-8800 Thalwil (CH); LANGLAIS, Vero, CH-8805 Richterswil (CH); SCHLITTLER, Räto, R., CH-8824 Schoenenberg (CH)
(74) Representative: Klett, Peter Michael
(86) International application number: PCT/IB1998/001445
(87) International publication number: WO 1999/016106

(56) References cited:
- SMOLYANINOV I I: "Photon emission from a layer of copper phthalocyanine molecules on a gold (111) film surface induced by STM" SURF SCI;SURFACE SCIENCE AUG 10 1996 ELSEVIER SCIENCE B.V., AMSTERDAM, NETHERLANDS, vol. 364, no. 1, 10 August 1996, pages 79-88, XP002065726
- BERNDT R ET AL: "Sub-nanometer lateral resolution in photon emission from C60 molecules on Au(110)" PROCEEDINGS OF THE 13TH EUROPEAN CONFERENCE ON SURFACE SCIENCE;WARWICK, UK AUG 30-SEP 4 1993, vol. 307-09, no. 1-3 part B, 30 August 1993, pages 1033-1037, XP002065727 SURF SCI;SURFACE SCIENCE APR 20 1994 PUBL BY ELSEVIER SCIENCE PUBLISHERS B.V., AMSTERDAM, NETH cited in the application

## Description

The invention relates to an apparatus and method for creating light as defined in the appended claims. More particularly, it relates to an apparatus comprising two electrodes in tunneling distance with a molecule inbetween. The molecule comprises a first entity, also called central entity, and at least one second entity, also called peripheral entity, which electrically decouples the central entity of the molecule from the electrode it is situated upon. The term "electrically decouples" is to be understood as the function of serving as an element which helps to keep a distance between the central entity and the electrode, wherein the distance is bigger than it would be if the molecule was chemisorbed or physisorbed on the electrode. With other words, the interaction of the first entity which is electrically decoupled in the herein mentioned sense, is smaller than the interaction of a physisorbed or chemisorbed first entity with that electrode. This interaction is usually defined by the overlap of the orbitals of the first entity and the electrode, respectively.

### TECHNICAL FIELD AND BACKGROUND OF THE INVENTION

With scanning tunneling microscopes, the properties of a tunneling junction have been explored.

In the publication "Optical Spectroscopy and microscopy using scanning tunneling microscopy" by Gimzewski, Bemdt, Schlittler, Kinnon. Welland, Wong, Dumas, Syrikii, Salvan and Hallimaoui in Proc. NATO ARW on Near-Field Optics (SNOM), Besançon, France, Oct. 26-28, 1992, pp 333-340, the tunneling current between a metallic tip and a metallic or even semiconductive sample surface has been found to consist of an elastic electron tunneling process and an inelastic process which gives rise to photon emission from the tip-sample region.

More detailed information about a tunneling junction between a tip and a metallic substrate can be found in "Photon emission from STM: Concepts" by Gimzewski in Proc. NATO ARW on Photons and Local Probes, pp 189-208. The metal surface shows for a constant tunneling current a light emission intensity distribution which has several maxima starting from an onset voltage of about 3.5 V.

In the article "Sub-nanometer lateral resolution in photon emission from C₆₀ molecules on Au (110)" by Berndt, Gaisch, Schneider, Gimzewski, Reihl, Schlittler and Tschudy in Surface Science, pp 1033-1037, 1994, the tip of a scanning tunneling microscope is used as a local electron source for exciting photon emission from ordered monolayers of C₆₀ molecules on an Au (110) surface. The photon emission intensity from the molecules is approximately one third of the intensity observed from the underlying Au substrate. The close proximity of the tip to the sample induces localized plasmon modes which are characterized by a strong electric field in the cavity formed by tip and sample and thus interact strongly with the tunneling electrons. The photon emission from the Au substrate is strongly suppressed when tunneling through a C₆₀ molecule.

### OBJECT AND ADVANTAGES OF THE INVENTION

It is an object of the invention according to claim 1 to provide an apparatus which is able to create light with a higher efficiency than so-far known arrangements do.

The obtainable light emission efficiency exceeds by orders of magnitude the observable external efficiency of OLEDs and other known light-emitting devices such as porous silicon. It therefore offers a huge range of applications which require much light and/or may use only little power and/or may use only little space.

When the peripheral entity is bound to the central entity such that they are movable relatively to each other, the molecule is able to adapt itself to the cavity which is built by the electrodes with the tunneling junction inbetween. This adaptation is deemed to result in a better match between an intensity peak in the radiative transition frequency spectrum, particularly the II-II* transition frequency, and an intensity peak in the tip-induced plasmon modes frequency spectrum. The adaptation can take place as a smooth transition as well as as an oscillatory transition.

When the molecule is pinnable to the first electrode, it can be fixed inbetween the two electrodes by applying the corresponding pinning voltage. With this method, the application of the pinning voltage manages to pin a molecule out of a multitude of molecules which exist on the first electrode in a gasphase. It is also possible that by pinning, the molecule is forced into a conformation which is more suitable for creating the emission of light.

The molecule has to undergo no adaptation, when it has a radiative frequency, particularly a II-II* transition frequency, which at least approximately equals the frequency of an intensity peak in the spectrum of the cavity between the first electrode and the second electrode.

When the central entity is in an essentially planar conformation compared to the peripheral entities, the prerequisite of electrical decoupling is easier to be fulfilled, since only one distance between the central entity and the first electrode needs to be controlled.

When the first electrode comprises a crystalline substrate, the molecule can be pinned more easily or even stays in a horizontally fixed position more easily since the substrate provides natural binding sites, where the molecule can rest. It is also possible that the crystalline structure forces the molecule to rotate and/or change its shape to find the best epitaxial matching condition and/or a conformation which automatically equals or is near to the conformation which is needed for the molecule to emit light most efficiently.

### SUMMARY OF THE INVENTION

The invention relates to the use of a molecule as light emitter in an apparatus comprising two electrodes at a tunneling distance from each other. Such a tunneling distance may as well lie in the subnanometer range as above that range and is the distance which allows a tunneling current to flow between the electrodes.

The molecule is e.g. a tertiary-butyl substituted tetracycline with the tetracycline as the central entity and the tertiary butyls as the peripheral entities which are movable with respect to the central entity. The molecule has several stable or metastable conformations depending on the states of its entities. These states are determined by the inner binding forces of the molecule, i.e. between the peripheral entities and the central entity, and the binding forces between the entities and the environment. The molecule is situated preferably on a crystalline substrate which serves as one of the electrodes. Hence, the inner forces of the molecule and the forces towards the substrate determine the conformations. In a first conformation, the peripheral entities have a binding force towards the substrate which dominates over the force between the central entity and the substrate. With other words, the central entity is so far away from the substrate that the force between it and the substrate is weaker than the force between the substrate and the peripheral entities. The binding force between the peripheral entities and the substrate are however sufficiently weak that the molecule is not fixed in its horizontal position. It therefore floats around on the substrate surface at room temperature.

Another conformation is dominated by the force between the central entity and the substrate. The central entity is then near enough at the substrate that the binding force holds the central entity to the substrate. In this conformation, the molecule remains in its position, therefore it is also called the pinned conformation. The peripheral entities in this conformation are somehow distorted or bent or more generally moved from their equilibration position, i.e. the position which they had in the first conformation. The holding force between the central entity and the substrate is stronger than eventual restoring forces between the peripheral entities and the central entity which try to form the molecule back to the first conformation. The molecule is immobilized by the combined force between the substrate and the central entity and between the peripheral entities and the substrate.

The molecule can be switched between the two stable conformations. The switching is induced by electrical voltage but can also occur through mechanical energy. The switching is reversible. However, also irreversible switching is possible for selected molecule types.

When an electrical current is allowed to flow through the substrate, light emission occurs. The substrate may have a predetermined surface structure, namely for a crystalline substrate the crystalline plane in which it lies. Light emission occurs on various planes, such as the {111} and the {100} plane. Copper, gold or silver are exemplary substrate materials on which the effect can be seen. Other materials for the substrate, such as polycrystalline materials or amorphous materials work as well.

The tip of the STM builds an electromagnetic cavity towards the substrate. The peripheral entities function as spacers for the central entity towards the substrate. Tungsten or other metals can be chosen as material of the tip or as a coating of a nonconductive tip. Other tip materials can be chosen which may even facilitate the light emission.

The effect is understood as a synergistic process between the electromagnetic cavity and the properties of the molecule such as a symmetry and/or the arrangement of its energy levels/orbitals. The appropriate molecule has a conformational flexibility such as the Tetracycline used exemplarily. This means that a suitable molecule need not have two or more stable states but can have different nonstable conformations. It can even be of the type which changes conformation in time as a result of an interaction with the cavity.

The electronic and/or crystallographic structure of the substrate may also play a part in determining the color of the emitted light, hence its wavelength, respectively frequency. When the molecule rests on the substrate, its conformation is at least partly determined by the structure of the substrate. The distance between the central entity and the substrate is again directly determined by the conformation and hence so are the resulting electronic levels of the molecule.

The idea is to create light emission by providing a molecule on a substrate, the molecule being situated upon its peripheral entities, serving as spacers, on said substrate such that a central entity of the molecule is spaced apart from the substrate surface and providing an electrical current which flows between the electrodes. The current may flow at least partly through the molecule. The intensity of the emitted light is increased when the molecule has an inherent conformational flexibility. The current is provided by a tunneling tip which is preferably adjacent to the central entity. The flexibility of the molecule may be influenced by the substrate.

A possible arrangement is an at least bistable molecule which rests on a crystalline substrate in one of its conformations and which is provided with electrical current such that it switches back and forth between two of its conformations or it oscillates around a more or less stable conformation. The parameters pressure, humidity and temperature are seen as uncritical, except in the range where they would interfere with any normal tunneling process.

### DESCRIPTION OF THE DRAWINGS

Examples of the invention are depicted in the drawings and described in detail below by way of example. It is shown in
Fig. 1 an arrangement with a tip and a molecule on a substrate,
Fig. 2 a first type of molecule,
Fig. 3 a second type of molecule,
Fig. 4 a diagram showing the photon intensity as a function of voltage and tunneling current for one molecule.
Fig. 5 a representation used to describe the light emission phenomenon.

All the figures are for sake of clarity not shown in real dimensions, nor are the relations between the dimensions shown in a realistic scale.

### DETAILED DESCRIPTION OF THE INVENTION

In the following, the various exemplary embodiments of the invention are described.

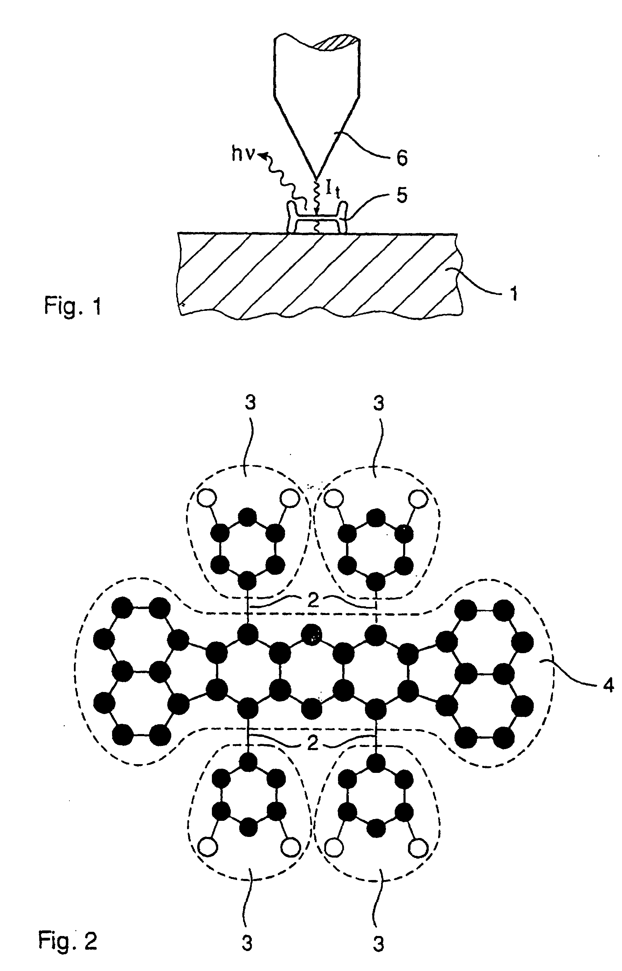

In figure 1, an arrangement for creating an emission of light is depicted. A first electrode in form of an electrically conductive substrate 1 carries a single molecule 5. Above the molecule 5, as a second electrode 6, a fine electrically conductive tip 6 is arranged at a distance that allows, in response to an operation voltage, electrons to tunnel between the tip 6 and the substrate 1, thereby creating a tunneling current Iₜ. The substrate 1 is an electrically conductive, crystalline material, such as a copper crystal cut in the {111} direction. When the tunneling current Iₜ is flowing, the molecule 5 emits light of energy hv. As an apparatus which provides a suited environment with the tip 6, a conventional STM (scanning tunneling microscope) can be used. The tip 6 may e.g. be made of or coated with Tungsten or any other conductive material for instance glass coated with gold.

In figure 2, the molecule 5 of figure 1 is depicted in more detail. The molecule 5 comprises a first entity, hereinafter called central entity 4, which here consists of hexagonal and pentagonal carbon aromatic rings. The carbon atoms are depicted as black disks in the drawing. The terminating H-atoms are not depicted for sake of simplicity. Bound to the central entity 4 are four second entities, hereinafter called peripheral entities 3, which consist of phenyl groups with tertiary-butyl attachments, hereinafter also referred to as t-butyl or t-Bu attachments. The t-butyl attachments are depicted as white disks in the drawing. The peripheral entities 3 are in the following also called "legs", since they represent the part of the molecule 5 which is in contact with the conductive substrate 1 and which can hold the central entity 4 apart from the surface of the substrate 1. The central entity 4 has a spatial structure that is here essentially planar compared to the legs 3 which are able to have various different positions relative to the central entity 4. These different positions are called conformations of the molecule 5. The molecule 5 can in some sense hence be compared to a table with movable legs. The change between two conformations with this molecule 5 is a smooth transition which has no abrupt processes and which is dependent on environmental influences.

The molecule 5 attaches to the substrate 1 in that it nucleates at a step edge or exists in a two-dimensional gas-like phase on a terrace. The gas-like phase is hereinafter defined as the first conformation. In this first conformation, the central entity 4 is positioned far enough away from the substrate surface that binding forces between the central entity 4 and the substrate 1 are negligible. The binding forces between the legs 3 and the substrate 1, however, keep the molecule 5 attached to the substrate 1, but only in the vertical direction. The molecule 5 remains horizontally movable and flows around on the substrate surface e.g. due to thermal excitation. This state is also called a two dimensional gas phase of the molecule 5.

A second conformation is defined in as the state of the molecule 5 when the central entity 4 comes close enough to the substrate 1 such that the binding forces between the central entity 4 and the substrate 1 increase significantly and effect a horizontal fixation of the molecule 5. This conformation is also called the "pinned state" of the molecule 5. In this state, however, the central entity 4 of the molecule 5 still remains electrically decoupled from the substrate 1, which means that its Π_{z}-orbitals do not strongly mix with the respective Π_{z}-orbitals of the substrate 1.

The central entity 4 of the molecule 5 would be in contrast hereto electrically coupled to the substrate 1 if it was chemisorbed or physisorbed on the substrate surface. The distance between the central entity 4 of the molecule 5 in the decoupled state is farther away than it would be if it was chemisorbed or physisorbed on the substrate 1. In other words, the interaction length between the central entity 4 and the substrate 1 is shorter in the decoupled state. This interaction length is the length of the overlap of the orbitals of the substrate 1 and of the central entity 4. Generally, the central entity 4 of the molecule 5 is in the decoupled state, when it is not chemisorbed or physisorbed on the substrate 1. The fact that the central entity 4 is decoupled does not mean that the peripheral entity 3 is also decoupled. The peripheral entity 3 can function as intermediate element which indirectly couples the central entity 4 to the substrate 1 e.g. in that it is chemisorbed and/or physisorbed at the substrate 1. The molecule 5 can then be regarded as electrically coupled to the substrate 1 although the central entity 4 is not directly coupled to the substrate 1. Hence in the herein mentioned sense, the central entity 4 is not directly electrically coupled to the substrate 1. hence not directly chemisorbed or physisorbed at the substrate 1.

Applying an electrical voltage above 2.5 V to the tunneling junction with the molecule 5 in it, brings the molecule 5 from the first conformation into the pinned state. This value of the operation voltage is called the pinning voltage. When being pinned out of a gas-phase of several molecules 5, the molecules 5 self-assemble themselves into two-dimensional islands on the substrate surface. The central entity 4 of the molecule 5 has also a distance from the tip 6 in which the molecule 5 is electrically decoupled from the tip 6.

The molecule 5 has more than the described two conformations. Particularly, since the molecule 5 has several legs 3, different positions of these legs 3 define further conformations. These conformations need not be stable but can also be of a type which only exists under the persisting influence of some external force. Even more, the conformations need not be discrete ones, which means that an uncountable number of such conformations may exist. In this case, the molecule 5 can best be described as being "flexible", i.e. its bonds can be distorted without breaking the molecule 5.

In the first conformation, the peripheral entities 3 of the molecule 5 are oriented normal to the plane of the central entity 4 and the central entity 4 is far enough away from the substrate surface to be only scarcely influenced by interaction forces. When e.g. pressed down by the tip 6, however, the central entity 4 gets so strongly attracted towards the substrate 1 due to attractive, here adhesive forces, that it remains in this second conformation even when the tip 6 is removed. This is a way to mechanically pin the molecule 5. Electrical pinning is also possible.

Via the tip 6, the tunneling current Iₜ flows through the molecule 5 at a given value of the operation voltage. The tunneling current Iₜ effects a change of conformation.

The molecule 5 is furthermore electronically excited by the tunneling electrons, such that the energy of the tunneling electrons is converted into a photon-emitting transition. This effects hence an electrically excited emission of light. It has been observed that this molecule 5 begins with photon emission after being pinned and that this photon emission then continues also when the operation voltage is reduced to levels below the pinning voltage. A maximum light-emission efficiency can be obtained with an operation voltage value of 2.3 V. This indicates that after an initial pinning, the molecule 5 also can be excited to light emission by voltages below the pinning voltage.

Since the light emission efficiency is much higher than with known molecules such as the C₆₀ molecule, a second effect is also influencing the light emission. This second effect is the enhancement of the light emission by the electromagnetic field between the tip 6 and the substrate 1. As is known, the tunneling current Iₜ over an empty tunneling junction creates tip-induced plasmon modes (localized electromagnetic modes) which have a predetermined frequency spectrum that shows a maximum of intensity and a decay in intensity around this maximum. The tip 6 together with the immediately adjacent part of the substrate surface and the gap inbetween forms a cavity, more precisely an electromagnetic cavity.

On the other hand, the molecule 5 has a radiative transition, namely here the Π-Π* transition which has a similar frequency spectrum which is predetermined by the molecule 5 and its actual conformation.

It is surmised that the matching between these two frequency distributions is important for the light emission intensity. Since the transition between the conformations of the molecule 5 is smooth, an automatic adaptation of the system may take place in that the molecule 5 changes its conformation again and again until its radiative transition frequency, here the Π-Π* transition frequency, matches an intensity peak in the tip-induced plasmon modes frequency spectrum. This state then seems to persist and also to yield the highest light-emission efficiency. Another possibility is a stable oscillation of conformation around this optimal state. It is also possible that the molecule 5 has more than one light-emitting states which may differ in the resulting light characteristics such as intensity or color.

It is one possible understanding that a suited molecule 5 which already has a radiative transition frequency, such as its Π-Π* transition frequency, matching a peak in the tip-induced plasmon modes frequency spectrum, needs neither a molecular flexibility nor a plurality of conformations for creating the light emission with comparable intensity.

The color spectrum of the emitted light lies for this molecule in the red to yellow spectral range and the light emanating from one single molecule 5 can be viewed by the human eye even in a lighted room and even with a tunneling current value of only 2 nA with an operation voltage value of 3V. This means that an input power of 6 x 10⁻⁹ W results in light visible with the naked eye. However, the molecule 5 is not limited to such low currents or voltages. The tunneling current can be raised even up to 1 µA resulting in a corresponding light emission. Higher voltage or current levels are certainly applicable.

The spectrum of the emitted light is assumed to be dependent on the conformation in which the molecule 5 emits light. A molecule 5 which oscillates around two or even more different conformations while emitting light hence might create different light wavelengths respectively colors.

The light emission works at room temperature and with pressures even higher than 10⁻⁴ HPa. Furthermore, the light-emission process is very stable. At least for the cited molecule types the process does not damage the molecule 5. Up to now, no time-related restriction was found. At a tunneling current value of 500 nA and an operation voltage value of 2.31 V, the light efficiency, defined as output power divided by input power has been estimated to be around 0.4 or even higher.

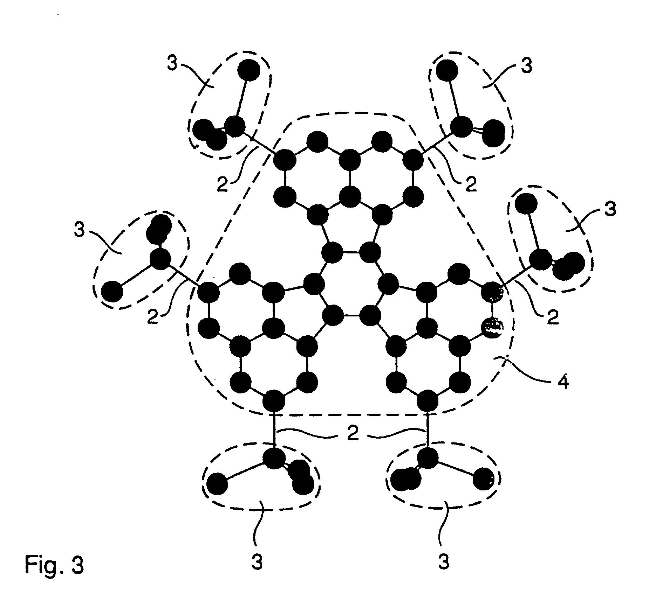

In figure 3, another type of the molecule 5 is depicted which also comprises a central entity 4 and here further comprises six peripheral entities 3 or legs. The molecule 5 is here a t-butyl-substituted tetracycline. The central entity 4 comprises again several pentagonal and hexagonal carbon rings, whereas the legs 3 consist of sp³ hybridized hydrocarbons.

The molecule 5 attaches to the substrate 1 in a similar way as does the molecule 5 from fig. 2. It also has the gas-like phase, defined as the first conformation. In this first conformation, the central entity 4 is again positioned far enough away from the substrate surface that binding forces between the central entity 4 and the substrate 1 are negligible. The molecule 5 remains horizontally movable and flows around e.g. due to thermal excitation.

The second conformation is again defined in that the central entity 4 comes closer to the substrate 1 such that the binding forces between the central entity 4 and the substrate 1 increase significantly and effect a horizontal fixation of the molecule 5. This is also called the "pinned state" of the molecule 5.

The distance between the central entity 4 of the molecule 5 in the decoupled state is farther away than it would be if it was chemisorbed or physisorbed on the substrate 1. Depending on environmental conditions, the molecule 5 can switch between these conformations. This particular molecule 5 changes between two conformations when an electrical voltage is applied.

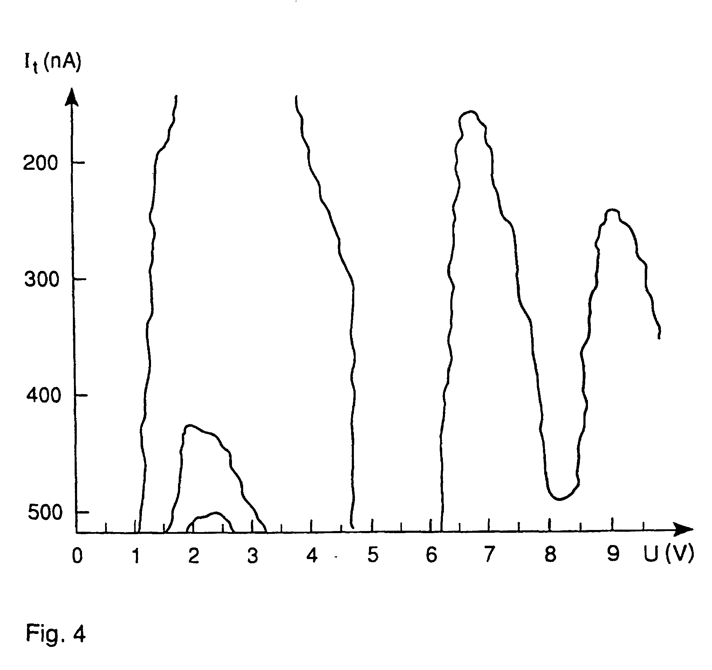

In figure 4, the dependence of light emission on voltage and current is shown in a diagram. The lines show levels of constant intensity, at arbitrary units. The light emission is, once the emission has started, observed to be linearly dependent on the tunneling current Iₜ and exhibits a maximum in terms of the applied operation voltage at around 2.21 V. The dependence on the operation voltage shows a first peak or maximum at around 3V, a second maximum at around 5V and a third maximum at around 9V. An equivalent peak spectrum can be observed with a clean metal substrate surface and the tip 6, albeit with much lower efficiency.

The first maximum is a global maximum and corresponds to a situation where the electric field strength is highest in the tunnel junction. The second and the third maximum correspond to the situation where electrons tunnel via radiating decay inelastically into empty Gundlach states as final state, whereby photons are emitted. These Gundlach states are mixed into the molecular states of the molecule 5 and the light intensity is higher than with a clean metal substrate 1. The observation of these resonances in the light emission shows that the scheme for tip-induced confined electromagnetic modes and their coupling with tunneling electrons is occurring. The shift in the maximum from 3.5V to 2.2V and the remarkable increase in the intensity are an evidence that the observed effect involves a synergistic coupling of the cavity and the molecular properties.

The observed physical effect is understood to ground on the fact that the central entity 4 of the molecule 5 is electrically decoupled from the electrically conductive substrate 1 by its legs 3. The term "electrically decoupled" is herein understood as the state when the central entity 4 of the molecule 5 is not physisorbed or chemisorbed on the substrate 1.

The molecular flexibility or existence of several conformations hereto contributes in that the molecule 5 can be adapted to its environment, namely the cavity and its frequency spectrum, by changing its molecular conformation. This adaptation is suggested to happen automatically when the molecule 5 is excited with tunneling electrons.

The described examples of the molecule 5 with several conformations comprise as the central entity 4 aromatic conjugated carbon-based sp²-delocalized rings and as the legs 3 sp³-hybridized hydrocarbons.

The molecule 5 can exist in different conformations which are characterized by the metastable orientations and/or positions of the different entities which it is made up of. The different entities 3, 4 of the molecule 5 consist e.g. of individual atoms or molecule-like sub-entities made up of atoms which are more strongly bound among each other than to the atoms of the other entities. The connections between individual entities 3, 4 may be single molecular bonds which can act as axis for a relative rotational motion of the entities 3, 4. Switching between the different conformations may comprise rotational realignment of the entities but also any other movement.

Appropriate combination of different types of the entities 3, 4 allows to design the molecule 5 such that it fulfills further requirements of a certain application in one conformation but are greatly different in another. Among the technically important properties which may undergo such variations are chemical activity, electrical conductivity, color, molecular dimensions, and the strength of adhesion to the substrate 1. Conversely, these changes can be used to identify the conformation of the molecule 5 by a variety of interrogating techniques. When the different conformations of the molecule 5 are defined as the minima of the potential energy of the system molecule/substrate with respect to its configurational coordinates, the conformations represent stable states of the molecule 5. It is then possible to define a relevant configurational coordinate for each transition between two conformations, such that the potential energy, when plotted as a function of this coordinate, has at least two characteristic minima. The position and depth of these minima determine the structural arrangement of the entities 3, 4 in these conformations and their stability with respect to thermal excitation and/or external influences, respectively. The deepest minimum then corresponds to the most stable conformation of the molecule. The other conformations are metastable. The molecule 5 can exist in a metastable conformation for indefinite time if the energy barriers between the corresponding energy minimum and its neighbors are large enough compared to the thermal energy which amounts to 25 meV at room temperature.

External influences which can provoke switching, or more generally a change of conformation, are, for instance, a mechanical force which deforms the molecule 5 to such an extent that it can snap into a different conformation like a macroscopic switch, irradiation with light or electrons that raises it into an excited state from which it can decay into the groundstate of another conformation, application of an electric field which lowers the height and/or width of a particular energy barrier to such an extent that the molecule 5 flips into another conformation due to thermal excitation or tunneling.

The molecule 5 can be synthesized using existing methods. When the position of the molecule 5 on the substrate 1 is fixed in at least one of the conformations, the immobilization of this molecule 5 at the substrate surface enables the use of micro- and nano-fabrication tools and methods for optional further processing.

The operation voltage can be direct as well as alternating and even go up to very high frequencies. To realize a electroluminescent device, such as a display, with a huge number of the molecules 5 embedded in corresponding cavities, it is possible to cover small metal particles with a number of the molecules 5 and then assembling the covered particles to one body in a transparent conductive material, like ITO (Indium Tin Oxide).

With the arrangement, displays can be realized that have reduced weight and power requirement. Flexible displays thinner than paper are possible. Integration of displays in spectacles or contact lenses is possible. This opens the field of displaying images directly on the retina or even amplifying a picture or making an infrared picture and displaying it directly in the eye, e.g. for people with a weak vision. Displaying plans or other information like in a head-up display for drivers is also possible in such a contact lens or eyeglass. The whole field of virtual reality can be easily realized with this in-eye display. Confidential information can also be displayed without disclosure problems. Since the light efficiency is so high, extremely low energy is dissipated in form of heat. The molecule 5 can be used as a very small and cold light source, e.g. in an endoscope. It is also imaginable that a small battery is combined with the molecule 5 as a nanotorch and is swallowable e.g. for medical examination.

For effecting the tunneling current, the apparatus comprises here voltage application means for applying an electrical voltage such that a tunneling current is flowing through the molecule 5. Any other means is also usable for effecting the tunneling current.

Hence the invention concerns a new form of electroluminescence from the molecules 5 induced by tunneling electrons. In the following, another approach for describing the effect of induced light emission is given.

Confined in a resonant electromagnetic cavity, the one or more individual molecules 5 emit photons in the visible and infrared range with a very high emission efficiency through localized excitation. Fine features, observed in the optical emission spectra are assigned to vibrational excited states. The emission mechanism can be described in terms of inelastic tunneling excitation of localized-electromagnetic modes between the preferably metallic tip 6 and the substrate surface. These modes resonantly promote electrons via Frank-Condon transitions in the molecule 5 which then radiatively decay. The results indicate an original method for sensitive vibrational spectroscopy on single molecules 5 at room temperature and also provide new concepts for using tunnel cavities as waveguides for communication on the nanometer scale.

For comparison, a view to known mechanisms is here given: A scanning tunneling microscope (STM) tip tunneling on noble-metal surfaces (Ag, Au, and Cu) behaves as a local light source with external quantum efficiencies of up to 0.1 % per tunneling electron. On C₆₀ adsorbed on Au( 110) surfaces, photons were spatially resolved but with lower intensity than bare gold itself. Electronic coupling of C₆₀ with the Au is sufficient to broaden substantially and lift the degeneracy of the highest occupied and lowest unoccupied molecular orbitals (HOMO and LUMO levels). In contrast, the fluorescence of molecules is strongly quenched by nonradiative damping in close proximity to a metal. This dissipation extends from a separation of 1 nm down to 3 nm from the metal surface.

The invention covers efficient light emission induced by tunneling electrons, from a class of molecules 5 which, by virtue of their architectures, are electronically decoupled from the tunneling electrodes 6, 1. They are observed to produce emission in the visible-infrared range with an estimated external quantum efficiency of up to 40\% per electron, even in sub-monolayer films. A continuous light emission with colors from red, orange, yellow, green and blue depending upon the excitation voltage, can be observed even with the naked eye under ambient illumination conditions. To put this efficiency in perspective, the highest external electroluminescence efficiencies from organic light-emitting diodes (OLEDs) have been measured to be 3% in the red range, 2% in the blue range, and from porous silicon 0.1-0.2%. The results indicate that the molecules 5 with specific molecular architectures can be used as high-efficiency light sources. The optical spectra are characterized by a family of sharp emission features, which are interpreted as fine vibrational structure arising from Frank-Condon transitions. This enables that an STM coupled with an optical spectrometer can be used at room temperature to locally probe molecular vibrational states.

Experiments were conducted under ultrahigh vacuum conditions at room temperature using an STM specifically designed for the observation of the light emission. Atomically clean substrates of Cu(100) and Ag( 110) single crystals were prepared by sputter-anneal cycles. Electrochemically etched tungsten tips cleaned by sputtering were used to induce electroluminescence. C₅₄H₆₆ molecules 5, subsequently called (HB-DC) for hexa-butyl decacyclene, were deposited by sublimation from a Knudsen cell onto metal surfaces heated to a temperature of 500 K to activate thermal diffusion for epitaxial growth. Optical spectra were recorded using a spectrometer (Jarrel Ash) equipped with a blazed holographic grating and a cooled optical multichannel analyzer. Light was collected from the STM using a f = 20.5mm lens which focused the light into an optical fiber cable from a solid angle of 0.1 steradian which was attached to the entrance slit of the spectrometer. Optical spectra from the HB-DC molecules 5 deposited on Ag(110) after background subtraction and normalization with respect to the tunneling current and the exposure time exhibit a family of sharp peaks where position is invariant with respect to Vₜ. Their intensity ratios vary with the applied bias voltages. Comparison with optical spectra obtained on a clean silver single crystal clearly indicate that the sharp features arise from molecular excited states, whereas the broad background, with a tail extending far into the long-wavelength range, resembles that of clean Ag. The invariance of the peaks position with Vₜ and the high quantum efficiency are characteristic of a resonant process. The intensity of each peak as a function of Vₜ exhibits oscillations independent of the wavelength. The peaks have a full width at half maximum (FWHM) of ~120meV and a constant energy splitting ΔE_{vib} of ~100meV. Using the Frank-Condon principle, the vibrational E_{vib} energy can often be approximated with a harmonic oscillator model for a simple molecule 5. E_{vib} is then given by E_{vib} = hω_{vib} (ν+1/2), where ω_{vib} is the vibration frequency and ν = 0, 1, 2, ... is the vibrational quantum number. The calculated vibrational frequency of ω_{vib} ~ 800cm⁻¹ is consistent with the classical vibrational modes of aromatic cycles.

The photon intensity recorded at monolayer coverage on Cu(100) plotted with respect to wavelength for various bias voltages shows, in contrast to the spectra taken with Ag(110) as substrate, a sharp cutoff of the light at 550nm (2.25 eV). This corresponds well to an interband transition in copper L₃(Q^{t}} -> E_{f}(L'₂) consistent with absorption of higher energy modes.

To obtain details on the relationship between the Vₜ and Iₜ on the photon intensity, Fig. 4 shows the map of photon intensity and tip retraction as a function of U=Vₜ and Iₜ This map shows an approximately linear increase in photon intensity with the tunneling current Iₜ. In contrast, with respect to Vₜ, over the range 0.3 to 30 nA a series of nodes and antinodes are observed. These intensity oscillations are identical in character to those recorded from a clean substrate and are assigned to field emission resonances as demonstrated by earlier photon-emission studies on clean noble metals. In that case, inelastically tunneling electrons excite electromagnetic modes located in the cavity between the electrodes 1, 6. The field emission resonances reflect the variations in coupling efficiency between the tunneling electrons and the resonant modes. The spatial extension of these localized electromagnetic modes is ~5nm. Consequently within the framework of the dielectric properties of the molecules 5 in close proximity to the junction will influence not only the modes themselves but also the inelastic tunneling probability of an electron exciting the modes.

These experimental results, indicate two possible mechanisms: First a direct inelastic tunneling excitation of the molecules 5 in a double barrier process mediated by the molecules 5 in the junction. In this scheme, a specific alignment of the molecule's electronic levels with the metal bands is supposed. Or second, inelastic tunneling between the electrodes 1, 6 excites the electromagnetic modes which couple to an indirect molecular excitation. The second model invokes a single tunnel barrier which is considered more favorable for the following reasons. The STM tip 6 is closer to metal and consequently the strength of the electromagnetic modes is enhanced. Combining the dielectric function of vacumm by that of the molecules 5 will change the light emission as evidenced by the sharp spectral features. Moreover, this single tunnel barrier process is consistent with a hysteresis effect which was observed in the experiment. Above a threshold Vₜ of ~3.5 V intense light emission was observed which then prevails down to ~2.5 V. This hysteresis is consistent with the removal of molecules 5 located directly in the local tunnelling electrons path by inelastic processes. Nevertheless, the molecules 5 remain in close proximity to the cavity defined between the tip 6 and the metal substrate 1, and thereby experience a high electromagnetic field.

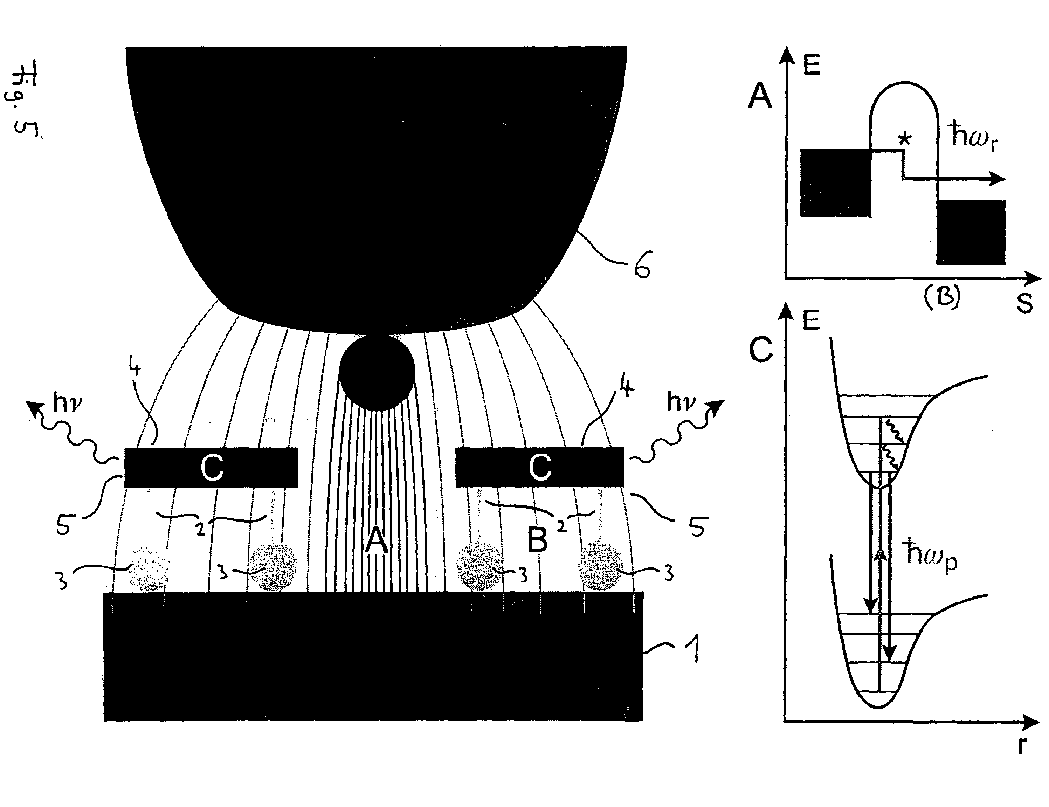

Figure 5 schematically represents the proposed mechanism that describes the light emission phenomenon in terms of a single tunnel barrier involving resonant coupling between electromagnetic modes and molecular vibrations. Electrons that tunnel inelastically (A) excite spatially localized electromagnetic modes hωᵣ (B) between the electrodes. The spatial extension of these modes (~5 nm) facilitates the electronic excitation of the molecules 5 that are decoupled from the electrodes but that remain in close proximity to the tunnel junction. Electromagnetic excitation of the molecules 5 occurs via resonant coupling with localized electromagnetic modes and in this case, the molecules 5 are then left in a excited state with an occupied LUMO and a hole in the HOMO. In this excited state thermal vibrations decay and emit radiatively via Frank-Condon transitions (C). The photon emission depends on the dielectric properties of both electrodes and the tunnel barrier region. Here the field enhancement is dominated by the dielectric properties of the metallic substrate and the proximity of the molecules 5 to the tunneling junction. In contrast to fluorescense from a molecule 5 on a metal surface, the excitation of the metal electrodes in STM prevents the quenching of the light via an energy transfer to the bulk and surface modes. The molecules 5 hence can be arranged such that only some part of or even no tunneling current flows though them, only taking advantage of the fact that the tunneling current induces or effects the light emission from the arrangement respectively the molecules 5. The regular case might be that the molecules 5 are situated in the vicinity of, i.e. adjacent to the tunneling junction, such that the main tunneling current flows directly between the tip 6 and the substrate 1 and only some electrons flow via the molecules 5.

In conclusion, these results demonstrate that electromagnetic molecules 5 are an efficient light source requiring little input power. Additional experiments conducted with other types of molecules 5 with similar spaces were observed to produce different Frank-Condon features. By combining STM with an optical spectrometer, a new method for vibrational spectroscopy of molecules 5 is proposed. Beyond potential applications such as flat-display technology and point sources of photons, the activation of localized electromagnetic modes constitutes an innovative and promising concept for wireless communication at the nanometer scale. The incorporation of more than one type of molecule 5 that give specific responses upon excitation can be regarded as a new method for the input and output of electronic signals using localized electromagnetic modes as a communication medium.

## Claims

1. Apparatus for creating a light emission, **characterized in that** at least one molecule (5) is positioned on a first electrode (1) which is located at a tunneling distance from a second electrode (6) being formed as a tip, said molecule (5) having a conformational flexibility and comprising a first entity (4) which is bound to at least one second entity (3) which decouples said first entity (4) from said first electrode (1) such that said first entity (4) is not chemisorbed or physisorbed at said first electrode (1) and **in that** said light emission is effectable through a tunneling current between said electrodes (1, 6).

2. Apparatus according to claim 1, **characterized in that** said molecule (5) is subjectable at least partly to the tunneling current.

3. Apparatus according to claim 1 or 2, **characterized in that** the second entity (3) is movable relatively to the first entity (4).

4. Apparatus according to one of claims 1 to 3, **characterized in that** the molecule (5) is pinnable to the first electrode (1) **in that** the first entity (4) is approached to said first electrode (1) such that the binding energy between said molecule (5) and said first electrode (1) is higher than the thermal energy at room temperature.

5. Apparatus according to one of claims 1 to 4, **characterized in that** the molecule (5) has a radiative transition frequency spectrum which comprises a local intensity maximum whose frequency, such as a Π-Π* transition frequency, at least approximately equals the frequency of a local intensity maximum in the frequency spectrum of the cavity between the first electrode (1) and the second electrode (6).

6. Apparatus according to one of claims 1 to 5, **characterized in that** the second entity (3) is located at the border of the first entity (4).

7. Apparatus according to one of claims 1 to 6, **characterized in that** the first entity (4) is in an essentially planar conformation compared to the second entity (3).

8. Apparatus according to one of claims 1 to 7, **characterized in that** the first electrode (1) comprises a crystalline substrate.

9. Method for creating a light emission, **characterized in that** at least one molecule (5) is positioned at a first electrode (1) which is located at a tunneling distance from a second electrode (6) being formed as a tip, said molecule (5) having a conformational flexibility and comprising a first entity (4) which is attached to at least one second entity (3) which decouples said first entity (4) from said first electrode (1) such that said first entity (4) is not chemisorbed or physisorbed at said first electrode (1) and **in that** said light emission is effected through a tunneling current between said electrodes (1,6) thereby switching said molecule (5) between different conformations.

10. Method according to claim 9, wherein said molecule (5) is subjected at least partly to the tunneling current.

## Patentansprüche

1. Vorrichtung zum Erzeugen einer Lichtemission, **dadurch gekennzeichnet, dass** mindestens ein Molekül (5) an einer ersten Elektrode (1) positioniert ist, welche in einem Tunnelabstand von einer zweiten als Spitze geformten Elektrode (6) angeordnet ist, wobei das Molekül (5) eine Konformationsflexibilität aufweist und eine erste Einheit (4) umfasst, die an mindestens eine zweite Einheit (3) gebunden ist, welche die erste Einheit (4) von der ersten Elektrode (1) so entkoppelt, dass die erste Einheit (4) an der ersten Elektrode (1) nicht chemisorbiert oder physisorbiert ist und dass die Lichtemission durch einen Tunnelstrom zwischen den Elektroden (1, 6) bewirkbar ist.

2. Vorrichtung nach Anspruch 1, **dadurch gekennzeichnet, dass** das Molekül (5) zumindest teilweise dem Tunnelstrom ausgesetzt werden kann.

3. Vorrichtung nach Anspruch 1 oder 2, **dadurch gekennzeichnet, dass** die zweite Einheit (3) gegenüber der ersten Einheit (4) beweglich ist.

4. Vorrichtung nach einem der Ansprüche 1 bis 3, **dadurch gekennzeichnet, dass** das Molekül (5) in der Weise an der ersten Elektrode (1) fixiert werden kann, dass die erste Einheit (4) der ersten Elektrode (1) so weit angenähert wird, dass die Bindungsenergie zwischen dem Molekül (5) und der ersten Elektrode (1) höher ist als die thermische Energie bei Raumtemperatur.

5. Vorrichtung nach einem der Ansprüche 1 bis 4, **dadurch gekennzeichnet, dass** das Molekül (5) ein Frequenzspektrum strahlender Übergänge hat, welches ein lokales Intensitätsmaximum aufweist, dessen Frequenz, wie zum Beispiel eine Π-Π*-Übergangsfrequenz, zumindest annähernd gleich der Frequenz eines lokalen Intensitätsmaximums im Frequenzspektrum des Hohlraums zwischen der ersten Elektrode (1) und der zweiten Elektrode (6) ist.

6. Vorrichtung nach einem der Ansprüche 1 bis 5, **dadurch gekennzeichnet, dass** die zweite Einheit (3) am Rand der ersten Einheit (4) angeordnet ist.

7. Vorrichtung nach einem der Ansprüche 1 bis 6, **dadurch gekennzeichnet, dass** sich die erste Einheit (4) in einer im Vergleich zur zweiten Einheit (3) im Wesentlichen planaren Konformation befindet.

8. Vorrichtung nach einem der Ansprüche 1 bis 7, **dadurch gekennzeichnet, dass** die erste Elektrode (1) ein kristallines Substrat umfasst.

9. Verfahren zum Erzeugen einer Lichtemission, **dadurch gekennzeichnet, dass** mindestens ein Molekül (5) an einer ersten Elektrode (1) positioniert wird, welche in einem Tunnelabstand von einer als Spitze geformten zweiten Elektrode (6) angeordnet ist, wobei das Molekül (5) eine Konformationsflexibilität aufweist und eine erste Einheit (4) umfasst, die an mindestens eine zweite Einheit (3) gebunden ist, welche die erste Einheit (4) von der ersten Elektrode (1) so entkoppelt, dass die erste Einheit (4) an der ersten Elektrode (1) nicht chemisorbiert oder physisorbiert ist und dass die Lichtemission durch einen Tunnelstrom zwischen den Elektroden (1, 6) bewirkt wird, wodurch das Molekül (5) zwischen verschiedenen Konformationen geschaltet wird.

10. Verfahren nach Anspruch 9, wobei das Molekül (5) dem Tunnelstrom zumindest teilweise ausgesetzt wird.

## Revendications

1. Dispositif destiné à créer une émission de lumière, **caractérisé en ce qu'**au moins une molécule (5) est positionnée sur une première électrode (1) qui est située à une distance d'effet tunnel par rapport à une seconde électrode (6) qui est formée en une pointe, ladite molécule (5) présentant une souplesse de conformation et comprenant une première entité (4) qui est liée à au moins une seconde entité (3) qui découple ladite première entité (4) de ladite première électrode (1) de manière à ce que ladite première entité (4) ne soit pas absorbée chimiquement ou physiquement à ladite première électrode (1) et **en ce que** ladite émission de lumière peut être activée grâce à un courant d'effet tunnel entre lesdites électrodes (1, 6).

2. Dispositif selon la revendication 1, **caractérisé en ce que** ladite molécule (5) peut être soumise au moins partiellement au courant d'effet tunnel.

3. Dispositif selon la revendication 1 ou 2, **caractérisé en ce que** la seconde entité (3) est mobile par rapport à la première entité (4).

4. Dispositif selon l'une des revendications 1 à 3, **caractérisé en ce que** la molécule (5) peut être immobilisée sur la première électrode (1) **en ce que** la première entité (4) est rapprochée de ladite première électrode (1) de telle manière que l'énergie de liaison entre ladite molécule (5) et ladite première électrode (1) soit supérieure à l'énergie thermique à température ambiante.

5. Dispositif selon l'une des revendications 1 à 4, **caractérisé en ce que** la molécule (5) présente un spectre de fréquences de transition de rayonnement qui comprend un maximum d'intensité local dont la fréquence, telle qu'une fréquence de transition Π-Π*, soit au moins approximativement égale à la fréquence d'un maximum d'intensité local du spectre de fréquences de la cavité entre la première électrode (1) et la seconde électrode (6).

6. Dispositif selon l'une des revendications 1 à 5, **caractérisé en ce que** la seconde entité (3) est située à la bordure de la première entité (4).

7. Dispositif selon l'une des revendications 1 à 6, **caractérisé en ce que** la première entité (4) est selon une conformation globalement plane par comparaison à la seconde entité (3).

8. Dispositif selon l'une des revendications 1 à 7, **caractérisé en ce que** la première électrode (1) comprend un substrat cristallin.

9. Procédé destiné à créer une émission de lumière, **caractérisé en ce qu'**au moins une molécule (5) est positionnée au niveau d'une première électrode (1) qui est située à une distance d'effet tunnel par rapport à une seconde électrode (6) qui est formée en une pointe, ladite molécule (5) présentant une souplesse de conformation et comprenant une première entité (4) qui est fixée au moins à une seconde entité (3) qui découple ladite première entité (4) de ladite première électrode (1) de manière à ce que ladite première entité (4) ne soit pas absorbée chimiquement ou physiquement à ladite première électrode (1) et **en ce que** l'émission de lumière est activée par l'intermédiaire d'un courant d'effet tunnel entre lesdites électrodes (1, 6) en faisant ainsi basculer ladite molécule (5) entre des conformations différentes.

10. Procédé selon la revendication 9, dans lequel ladite molécule (5) est soumise au moins partiellement au courant d'effet tunnel.
